# EUROPEAN PATENT SPECIFICATION

(11) **EP 2 943 166 B1**
(45) Date of publication and mention of the grant of the patent: **05.08.2020**
(21) Application number: 13830131.2
(22) Date of filing: 20.12.2013
(51) Int. Cl.: A61F 2/68, A61F 2/80

(54) **A FOOT WITH A VACUUM UNIT ACTIVATED BY AN ANKLE MOTION**
FUSS MIT EINER DURCH EINE FUSSKNÖCHELBEWEGUNG AKTIVIERTEN VAKUUMEINRICHTUNG
PIED À POMPE D'ASPIRATION ACTIONNÉE PAR UN MOUVEMENT DE LA CHEVILLE

(30) Priority: 10.01.2013 TR 201300341; 02.08.2013 TR 201309443
(43) Date of publication of application: 18.11.2015
(73) Proprietor: Ortotek Ortopedi Protez Rehabilitasyon Merkezi Ticaret Limited Sirketi, 06000 Ankara (TR)
(72) Inventor: DUGER, Mustafa, 06000 Ankara (TR)
(74) Representative: Berkkam, Ayfer
(86) International application number: PCT/TR2013/000370
(87) International publication number: WO 2014/109723

(56) References cited:
- EP-A1- 0 482 809
- WO-A2-02/067825
- DE-B- 1 002 504

## Description

### Technical Field

The present invention is related to a foot with a vacuum unit activated by an ankle motion which can suck the air out of the carrier shell by using the displacement established by the transformation of the angular motion into linear motion which is formed between the tibia axis and foot sole plane of prosthesis legs during walking.

### Prior Art

Patients, who have been amputated from their members due to various reasons, use various types of prosthesis in order to either increase their living standards or to adapt to social life. Said prosthesis, are prepared with care regarding their appearance and functionality in order to be able to replace their real members cosmetically and functionally. Although it is possible to homologise these prosthesis's cosmetically it is actually not possible for prosthesis to completely replace a member in terms of functionality.

However the prosthesis parts obtained in the studies carried out parallel with technological developments, have enabled to reach samples of prosthesis which are easier to use and more functional. One such development has been explained within the patent application numbered US2012143351 wherein it is aimed to prevent the prosthesis to be dislocated. In the related application a vacuum unit which sucks the air inside the socket (shell) into which the stump enters to and which is used in prosthesis placed under or above the knee is described. The dislocation caused by the air locked between the socket and the stump is eliminated by means of said vacuum unit. By eliminating this problem the patients can become an inseparable whole with their prosthesis and they do not feel the anxiety that their prosthesis will be dislocated.

A weight actuated vacuum pump for an artificial limb is disclosed in the document numbered WO02067825A2.

### Brief description of the Invention

The invention aims to develop a foot with a vacuum unit which enables to suck the air remaining between the sock worn and the stump or the stump and the socket of the prosthesis used by the patients using prosthesis above the knee or under the knee.

Another aim of the invention is to provide a vacuum which uses mechanical energy to drive the piston which actuates the vacuum rather than the need for electrical energy to drive it. Besides this another aim of the invention is to eliminate the necessity for a different/special motion as the piston motions within the vacuum medium triggers a force obtained from the natural walking actions.

The invention can be used in prosthesis that imitate the angular motion, during walking, obtained between the plane of the sole of the foot and the angle where the tibia bone is located in humans who have not lost a member. By means of the vacuum unit which converts angular motion into linear motion, wherein said vacuum unit being located on the piston within the prosthesis that imitate the angular motion between the tibia bone axis and foot sole plane, the motion of the casing is provided and by this means the air inside the volume between the sock worn over the stump or the socket and the stump is vacuumed. By this means it is aimed to decrease the movements of the stump of the prosthesis user inside the socket thus making the prosthesis usage easier. It is also enabled for the prosthesis connected to the stump to be able to move like a member connected to the body.

### Detailed description of the Invention

The foot with a vacuum unit activated by the ankle movement in order to reach the aims of the invention has been shown in the attached figures wherein said figures illustrate the following:

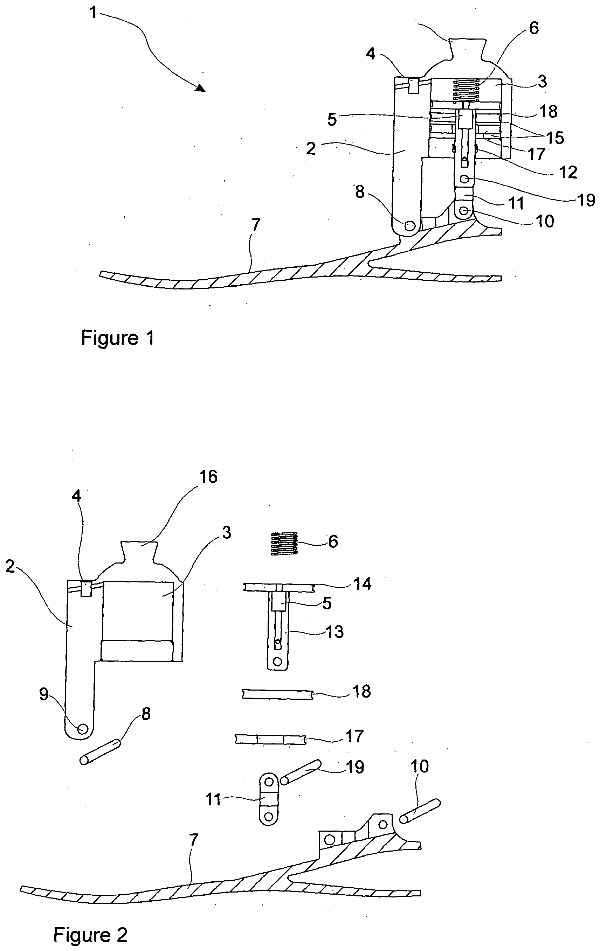

Figure 1 - Is the left side view of the hydraulic application of the foot with a vacuum unit activated by the ankle, subject to the invention.
Figure 2 - Is the dissected view of the hydraulic application of the foot with a vacuum unit activated by the ankle, subject to the invention.

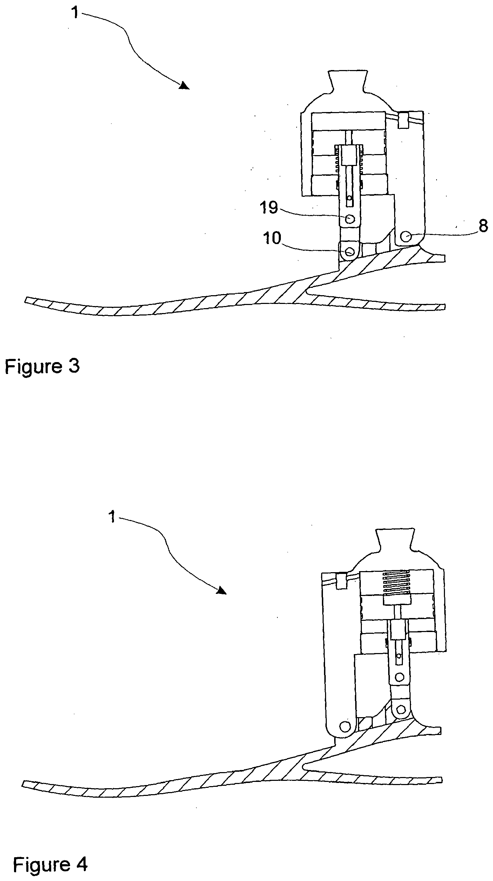

Figure 3 - Is the left side view of the foot with a vacuum unit activated by the ankle subject to the invention adapted such that the ankle shaft is closer to the heel in comparison to the body shaft.
Figure 4 - Is the left side view of the foot with a vacuum activated by the ankle subject to the invention adapted such that the body shaft is closer to the heel in comparison to the ankle shaft.

The parts in the figures have each been numbered and their references have been listed below.
1. Foot with vacuum unit
2. Body
3. Vacuum shell
4. Air inlet valve
5. Air outlet valve
6. Return spring
7. Foot sole
8. Ankle shaft
9. Ankle slot
10. Body shaft
11. Piston link
12. Impermeability means
13. Piston arm
14. Piston head
15. Hydraulic reservoir
16. Connection pyramid
17. Hydraulic reservoir separator
18. Hydraulic reservoir wall
19. Link pin
The foot (1) with a vacuum unit activated by ankle movement subject to the invention, basically comprises a body (2) located between the prosthesis leg and the sole of the foot (7) and a foot sole (7). The body (2) comprises a vacuum shell (3) which provides vacuum, an air inlet valve (4) which enables the air to reach the vacuum shell (3), an air outlet valve (5) which enables to discharge the air inside the vacuum shell (3), and a return spring (6) which ensures that taking a step shows a response close to a natural response and/or which ensures that the vacuum value is adjusted. Moreover an ankle shaft (8) used as the pivoting point in order to imitate the natural movement of the ankle, and an ankle slot (9) which enables connection to the ankle shaft (8), a body shaft (10) which converts this angular movement into linear movement, a piston link (11) connected to the body shaft (10), a piston arm (13) connected to this piston link (11)a piston head connected to said piston arm (13), and a prosthesis connection point (17) which ensures the connection to the body of the prosthesis (2) is present on the body (2). The body (2) is connected to the sole of the foot (7) by means of the body shaft (10), ankle shaft (8) and the ankle slot (9) into which said ankle shaft (8) can enter into. The piston link (11) is connected to the sole of the foot (7) by means of the body shaft (10) and to the piston arm (13) by means of the link pin (19).

In the hydraulic applications of the invention in order to imitate the natural walking motion within the body (2) more correctly, the hydraulic applications comprises: a hydraulic reservoir (15) which comprises hydraulic liquid, and which has a fixed volume, a hydraulic reservoir wall (18) which has a fixed location inside the body (2) which ensures that the hydraulic reservoir (15) stays at a fixed volume and various kinds of impermeability means (12) that ensure that the hydraulic liquid does not leak into the body (2). Moreover the hydraulic reservoir (15) has a mobile hydraulic reservoir separator (17) into which the hydraulic liquid can pass through in a controlled manner. By this means changes in the volume of the chambers within the two chamber structure of the hydraulic reservoir (15) can be carried out. The vacuum shell (3) is located at the top and the hydraulic reservoir (15) is located below it inside the body (2).

The foot (1) with a vacuum unit subject to the invention creates vacuum inside the socket of the leg wherein the prosthesis is attached to and it is used to ensure that the sock worn over the stump or the stump itself does not slip or slide inside the socket.

During a natural walking process the angle between the axis of the tibia bone and the floor plane changes. In addition, the angle between the floor plane and the plane of the sole of the foot (7) also changes in different values due to the angular change between the tibia bone axis and the floor plane. As a result an angular motion is established between the tibia bone axis and the plane of the foot sole (7).

In order to make sure that the socket to which the prosthesis is attached to do not slip from the leg and that the walking motion is carried out more properly, the leg prosthesis can be worn with silicon or fabric socks.

The vacuum unit (1) activated by the ankle movement subject to the invention, aims to be used in order to establish vacuum between the socket and the stump/or the sock worn over the stump by the linear motion created when the angular motion between the tibia bone axis and the plane of the sole of the foot (7) is imitated. By this means it is provided for the prosthesis user to move like a person moving naturally.

The invention, imitates the angular movement between the tibia bone axis and the foot sole (7) plane established during the foot swinging or step taking motions included in the natural walking motion, by taking the ankle shaft (8) as the pivoting point. During this motion where the pivoting point is the ankle shaft (8) the piston link (11) connected to the foot sole (7) converts the angular motion into linear motion via the piston arm (13). The piston arm (13) moving inside the body (2) with a linear motion, is counter supported with the return spring (6) and the hydraulic liquid located inside the fixed volume hydraulic reservoir (15) in order to preserve the nature of the motion. By this means a counter support in relation to the fast changes of the angle of the sole of the foot (7) of the prosthesis user and the body is created. This ensures that the balance of the user is not affected by the sudden load changes that could be created on the prosthesis by the user, during walking.

The angular motion between the foot sole (7) plane and the tibia bone axis, can be created during inertia when the foot is in the air during walking or more often during taking steps. The step taking process begins with the foot being lifted off the ground and when the heel of the foot is stepped on the ground the contact of the floor plane and the foot sole (7) is established. During this stage the angle between the tibia bone and the floor plane is decreased at the heel direction. The heel part of the foot sole (7) contacts the floor. At this moment the toe section of the foot sole (7) is in the air. During this stage of the movement a 90 degree angle is established between the foot sole (7) plane and the tibia bone axis. In the next stage of step taking the angle between the sole of the foot (7) and the floor plane decreases until the foot sole (7) is parallel to the floor plane. During this moment the angle between the foot sole (7) and the tibia bone axis increases. By means of the increase of the contact area of the foot sole (7) with the floor plane, a balance area is established for the body to lunge forward. Thus the body will lunge forward at a planar angle.

During this forward movement the angle that has increased between the tibia bone axis and the foot sole (7) again decreased to approximately 90 degrees. When the tibia bone axis is positioned such that it provides a right angle with the floor plane, the foot sole (7) is parallel to the floor plane. In the final stage of step taking, the angle left between the foot sole (7) heel section between the tibia bone axis and the floor plane increases. While the tibia bone axis leans forward the angle between the foot sole (7) decreases. Together with the inertia of the body which take support from the other foot that has moved forward, the heel section cuts off the contact with the floor plane such that the tip of the toes of the foot sole (7) are in contact with the floor plane. Finally the foot sole (7) cuts off the contact with the floor plane completely and it is pulled back by the knee in order to take a step forward and then it enters the swinging process.

The foot (1) with a vacuum unit activated by an ankle movement subject to the invention, has been aimed to create a vacuum so that the socket used in the prosthesis is used more comfortably by converting the angular movement created due to the angular changes between the tibia bone axis and the foot sole (7) in said step taking stages, into a linear motion by means of the piston links (11) and the piston arm (13).

The motion of the piston arm (13) inside the body (2) is counter supported with the return spring (6) in order to prevent the upsetting of user's balance. This return spring (6) that forms a counter support which can be changed by the user establishes counter support against the piston arm (13) which can have different rigidities in accordance with the requirements and demands of the user. By this means it is possible for the prosthesis user to imitate the natural walk of a person, without upsetting the balance of the user.

Due to this motion of the piston arm (13) inside the body (2), changes in the volume of the piston head (14) vacuum shell (3) occurs. During the motion of the piston head (14) toward the foot sole (7) plane the vacuum shell (3) volume expands and in the motion in the opposite direction the volume of the vacuum shell (3) decreases.

In the case that the volume of the vacuum shell (3) expands, the air inside the vacuum shell (3) spreads to more volume and the pressure is lowered; thus vacuum is formed.

The low pressure that is created in this vacuum medium, is used to draw air from the air inlet valve (4) which is a one way valve. By this means, air is drawn out of the area located between the stump or the sock worn over the stump and the socket, and a vacuum is created between the stump or the sock worn over the stump and the socket.

In the case that the volume of the vacuum shell (3) is decreased, the air drawn out of the air inlet valve (4) is found at a lower volume and thus high pressure is created. In this case the excess air is again discharged from the air outlet valve (5) which is again a single way valve. Thereby it is provided that the angular motion obtained from the ankle motion creates a vacuum between the socket and the stump or the sock worn over the stump and the socket such that the natural way of walking is not disrupted.

In the application of the invention, the volume between the body (2) periphery and the fixed position hydraulic reservoir wall (18) inside the body (2) constitutes the hydraulic reservoir (15). The hydraulic liquid inside the hydraulic reservoir (15) is located as separated into two chambers by means of the hydraulic reservoir separator (17). The hydraulic reservoir separator (17) provides the hydraulic liquid substitution in a controlled manner between the chambers which are located inside the hydraulic reservoir (15). As a result, this will function as a system that can provide a counter support together with the return spring (6) to prevent the disruption of the balance of sudden load changes embarked on the foot by the body during a walking motion. Moreover in this application, in order for the hydraulic liquid to stay inside the hydraulic reservoir (15) and in order to ensure that the hydraulic liquid is controlled between the chambers that have been separated into two, various impermeability means (12) shall be used.

The conversion of the angular motion between the foot sole (7) and the tibia bone axis, by means of the piston link (11) and the piston arm (13) attached to the piston link, can either be used for the orientation of hydraulic or air flow. The hydraulic motion is provided by decreasing the volume below the hydraulic reservoir (15) and increasing the volume above the reservoir. By this means it is enabled for the imitation of the natural foot swinging motion, to be soft and resistant against high pressure.

In the various applications of the vacuum unit foot (1) subject to the invention, the positioning of the ankle shaft (8) functioning as the ankle, and the slot (9) through which the ankle shaft (8) enters and the positioning of the body shaft (10) on the foot sole (7) could be different. In the application wherein the position of the body shaft (10) on the foot sole (7), is placed to be in front of the ankle shaft (8) the pivoting point of the angular motion between the ankle shaft (8) and consequently the tibia bone axis and the foot sole (7) will be positioned at the back when compared with a normal application and in order to balance the body weight the foot sole (7) can be extended from the heel level. Furthermore in this application the realization sequence of the vacuum process will also change. Following, placing the heel on the ground which is the initial part of the stepping process, the angle between the foot sole (7) and the tibia bone plane will increase and as a result at this moment the vacuum unit (1) will extract air from the inlet valve (4) and a vacuum will be formed inside the socket. In the following stages of the stepping process, when the angle between the foot sole (7) and the tibia bone decreased, the air inside the vacuum shell (3) is discharged via the air outlet valve (5).

The hydraulic adjustment can also be used in order to adjust the response of the hydraulic liquid of the hydraulic applications of the invention located inside the hydraulic reservoir (15) in accordance with the requirements and demands of the user. This adjustment screw can be used to increase or decrease the counter response on the piston arm (13) of the hydraulic reservoir (15).

Moreover in the hydraulic applications of the vacuum unit foot (1) with ankle activation subject to the invention, two adjustment systems must be present in order to determine the difficulty of the movement of the foot bilaterally both to the front and the back. These systems can be hydraulic, spring or screw systems. One of the adjustment systems shall help to adjust the difficulty of the motions where the foot sole (7) rotates clockwise with the ankle shaft (8) being the centre and shall help this motion to imitate the natural walking motion. Similarly the other adjustment system shall help to adjust the difficulty level of the motion where the foot sole (7) is rotated at a counter clockwise direction centric to the ankle shaft (8). In the hydraulic applications of the invention, three adjustment systems are present in order to adjust the two hydraulic motions and the one vacuum shell (3) motion. In systems which use air the number of said adjustment system is one.

In the hydraulic applications of the invention, the hydraulic reservoir (15) can be positioned to floor plane such that it is further away from the vacuum shell (3). In this application, the hydraulic reservoir (15) is located on the vacuum shell (3). Thereby using gravity the lubrication of the mobile elements of the vacuum shell (3) can also be provided.

The flow rate and the depth of the vacuum unit foot (1) subject to the invention, by means of the hydraulic liquid hydraulic reservoir separator (17) is adjusted and this will ensure that the speed of the upwards and downwards motion of the piston is controlled. Nevertheless the structure of the reservoir separator (17) can completely be eliminated and instead adjustment valve components can be used and the speed of the substitution of the fluid inside both reservoirs of the hydraulic reservoir (15) can be controlled. It is possible for such controls to be carried out by turning the adjustment valve by the user or the operator mechanically and it is also possible to control this flow by using an electronic control adjustment device. The electronic control device shall enable the adjustment of the speed of the angular motion to be carried out by the foot sole (7) in connection with the speed of walking of the patient, the weight of the patient and other similar variables.

## Claims

1. A vacuum unit foot (1) adapted to be connected to a prosthetic leg having a socket, the vacuum unit foot (1) being adapted to draw the air out of the socket by using the displacement established with the conversion of the angular motion into linear motion that has been created during the walking process between the tibia axis and the foot sole plane, the vacuum unit foot (1) comprising
- a foot sole (7)
- a body (2) located between the prosthesis leg and the foot sole (7),
- a vacuum shell (3) inside the body (2), in which vacuum is created during use,
- an inlet valve (4) which enables the air to be drawn into the vacuum shell (3),
- an air outlet valve (5) that enables air to be discharged from the vacuum shell (3),
- wherein the body (2) is adapted to move angularly around an ankle slot (9) which enables the connection of an ankle shaft (8) that is used as the pivoting point in order to imitate the natural motion of the ankle,
- a piston head (14) adapted to change the volume of the vacuum shell (3),
- a piston arm (13) which the piston head (14) is connected to,
- a return spring (6) that is adapted to counter support the piston arm (13)
**characterized in that** it further comprises a piston link (11) connected to the foot sole (7) by a body shaft (10) and the piston arm (13) by a link pin (19) wherein the piston link (11) is adapted to convert the angular motion of the foot sole (7) around the ankle shaft (8) into linear motion of the piston arm (13).

2. A vacuum unit foot (1) according to claim 1, **characterized in that** it comprises hydraulic liquid in order to imitate as closely possible the natural walking motion inside the body (2) where the vacuum shell (2) is located, and a fixed volume hydraulic reservoir (15), a hydraulic reservoir wall (18) having a fixed location inside the body (2) adapted to maintain the hydraulic reservoir (15) at a fixed volume, and a mobile hydraulic reservoir separator (17) separating the hydraulic reservoir (15) into two chambers wherein the hydraulic reservoir separator (17) is adapted to control the flow rate of the hydraulic liquid between said two chambers.

3. A vacuum unit foot (1) according to claim 2, **characterized in that** it comprises a vacuum shell (3) positioned above and hydraulic reservoir (15) positioned under it inside a body (2).

4. A vacuum unit foot (1) according to claim 3, **characterized in that** the hydraulic reservoir (15) that could ensure the lubrication of the moving elements of the vacuum shell (3) by means of gravity is located on the vacuum shell (3)

## Patentansprüche

1. Vakuumeinheitsfuß (1), der angepasst ist, um mit einer Beinprothese mit einer Pfanne verbunden zu werden, wobei der Vakuumeinheitsfuß (1) angepasst ist, um die Luft aus der Pfanne herauszuziehen, indem die Verschiebung verwendet wird, die bei der Umwandlung der Winkelbewegung in eine lineare Bewegung entsteht, die während des Gehvorgangs zwischen der Tibiaachse und der Fußsohlenebene erzeugt wurde, wobei der Vakuumeinheitsfuß (1) aufweist:
- eine Fußsohle (7),
- einem Körper (2), der sich zwischen dem Prothesenbein und der Fußsohle (7) befindet,
- eine Vakuumschale (3) im Inneren des Körpers (2), in der während des Gebrauchs ein Vakuum erzeugt wird,
- ein Einlassventil (4), das das Ansaugen der Luft in die Vakuumschale (3) ermöglicht,
- ein Luftauslassventil (5), das das Ablassen von Luft aus der Vakuumschale (3) ermöglicht,
- wobei der Körper (2) angepasst ist, um sich winkelig um einen Knöchelschlitz (9) zu bewegen, der die Verbindung eines Knöchelschafts (8) ermöglicht, der als Drehpunkt verwendet wird, um die natürliche Bewegung des Knöchels nachzuahmen,
- einen Kolbenkopf (14), der angepasst ist, um das Volumen der Vakuumschale (3) zu ändern,
- einen Kolbenarm (13), mit dem der Kolbenkopf (14) verbunden ist,
- eine Rückholfeder (6), die so angepasst ist, dass sie den Kolbenarm (13) gegenläufig unterstützt
**dadurch gekennzeichnet, dass** es ferner ein Kolbenglied (11) umfasst, das mit der Fußsohle (7) durch eine Körperwelle (10) und den Kolbenarm (13) durch einen Verbindungsstift (19) verbunden ist, wobei das Kolbenglied (11) angepasst ist um die Winkelbewegung der Fußsohle (7) um den Knöchelschaft (8) in eine lineare Bewegung des Kolbenarms (13) umzuwandeln.

2. Vakuumeinheitsfuß (1) nach Anspruch 1, **dadurch gekennzeichnet, dass** er eine Hydraulikflüssigkeit zur möglichst genauen Nachahmung der natürlichen Gehbewegung im Inneren des Körpers (2), wo sich die Vakuumschale (2) befindet, und einen Hydraulikbehälter (15) mit festem Volumen umfasst, eine Hydraulikbehälterwand (18) mit einer festen Stelle innerhalb des Körpers (2), die angepasst ist, um den Hydraulikbehälter (15) auf einem festen Volumen zu halten, und einen mobilen Hydraikbehälterabscheider (17), der den Hydraulikbehälter (15) in zwei Kammern trennt, wobei der Hydraulikbehälterabscheider (17) angepasst ist, um die Strömungsrate der Hydraulikflüssigkeit zwischen den beiden Kammern zu steuern.

3. Vakuumeinheitsfuß (1) nach Anspruch 2, **dadurch gekennzeichnet, dass** er eine darüber positionierte Vakuumschale (3) und einen darunter positionierten Hydraulikbehälter (15) innerhalb eines Körpers (2) umfasst.

4. Vakuumeinheitsfuß (1) nach Anspruch 3, **dadurch gekennzeichnet, dass** sich der Hydraulikbehälter (15), der die Schmierung der beweglichen Elemente der Vakuumschale (3) mittels Schwerkraft sicherstellen könnte, auf der Vakuumschale (3) befindet.

## Revendications

1. Pied de l'unité à vide (1) adapté pour être connecté à une jambe prothétique ayant une prise, le pied de l'unité à vide (1) étant adapté pour aspirer l'air hors de la prise en utilisant le déplacement établi avec la conversion du mouvement angulaire en mouvement linéaire qui a été créé pendant le processus de marche entre l'axe du tibia et le plan de la plante du pied, le pied de l'unité à vide (1) comprenant
- une plante du pied (7)
- un corps (2) situé entre la prothèse de la jambe et la plante du pied (7),
- une enveloppe sous vide (3) à l'intérieur du corps (2), dans laquelle le vide est créé lors de l'utilisation,
- une vanne d'admission (4) qui permet d'aspirer l'air dans l'enveloppe sous vide (3),
- une vanne de sortie d'air (5) qui permet d'évacuer l'air de l'enveloppe sous vide (3),
- dans laquelle le corps (2) est adapté pour se déplacer angulairement autour d'une fente de cheville (9) qui permet la connexion d'une tige de cheville (8) qui est utilisée comme point de pivotement afin d'imiter le mouvement naturel de la cheville,
- une tête de piston (14) adaptée pour modifier le volume de l'enveloppe sous vide (3),
- un bras de piston (13) auquel la tête de piston (14) est connectée,
- un ressort de rappel (6) qui est adapté pour contre-supporter le bras de piston (13)
**caractérisé en ce qu'**il comprend en outre une bielle de piston (11) connectée à la plante du pied (7) par une tige de corps (10) et au bras de piston (13) par un axe de liaison (19) dans lequel la bielle de piston (11) est adaptée pour convertir le mouvement angulaire de la plante du pied (7) autour de la tige de cheville (8) en un mouvement linéaire du bras de piston (13).

2. Pied de l'unité à vide (1) selon la revendication 1, **caractérisé en ce qu'**il comprend un liquide hydraulique afin d'imiter le plus étroitement possible le mouvement naturel de marche à l'intérieur du corps (2) où se trouve l'enveloppe sous vide (2), et un réservoir hydraulique (15) à volume fixe, une paroi de réservoir hydraulique (18) ayant un emplacement fixe à l'intérieur du corps (2) adapté pour maintenir le réservoir hydraulique (15) à un volume fixe, et un séparateur de réservoir hydraulique mobile (17) séparant le réservoir hydraulique (15) en deux chambres dans lequel le séparateur de réservoir hydraulique (17) est adapté pour contrôler le débit du liquide hydraulique entre lesdites deux chambres.

3. Pied de l'unité à vide (1) selon la revendication 2, **caractérisé en ce qu'**il comprend une enveloppe sous vide (3) placée au-dessus et un réservoir hydraulique (15) placé en dessous à l'intérieur d'un corps (2).

4. Pied de l'unité à vide (1) selon la revendication 3, **caractérisé en ce que** le réservoir hydraulique (15) qui pourrait assurer la lubrification des éléments mobiles de l'enveloppe sous vide (3) par gravité est situé sur l'enveloppe sous vide (3).
